# EUROPÄISCHE PATENTSCHRIFT

(11) **EP 0 879 300 B1**
(45) Veröffentlichungstag und Bekanntmachung des Hinweises auf die Patenterteilung: **18.12.2002**
(21) Anmeldenummer: 97904362.7
(22) Anmeldetag: 31.01.1997
(51) Int. Cl.: C12Q 1/68

(54) **VERWENDUNG VON PRIMERN FÜR UNIVERSELLE FINGERPRINT-ANALYSEN**
USE OF PRIMERS FOR UNIVERSAL FINGERPRINT ANALYSIS
UTILISATION D'AMORCES POUR ANALYSE UNIVERSELLE DES EMPREINTES DIGITALES D'ADN

(30) Priorität: 02.02.1996 EP 96101515; 19.09.1996 US 26912 P
(43) Veröffentlichungstag der Anmeldung: 25.11.1998
(73) Patentinhaber: MAX-PLANCK-GESELLSCHAFT ZUR FÖRDERUNG DER WISSENSCHAFTEN E.V., 14195 Berlin (DE)
(72) Erfinder: ROHDE, Wolfgang, D-35418 Busek (DE)
(74) Vertreter: VOSSIUS & PARTNER
(86) Internationale Anmeldenummer: EP9700442
(87) Internationale Veröffentlichungsnummer: WO97028278

(56) Entgegenhaltungen:
- EP-A- 0 433 748
- EP-A- 0 647 718
- WO-A-93/08297
- J. GENET. & BREED., Bd. 49, - 1995 Seiten 179-186, XP000677744 RHODE W. ET AL.,: "Genome analysis of Cocos nucifera L. by PCR amplification of spacer sequences separating a subset of copia-like EcoRI repetitive sequences" in der Anmeldung erwähnt
- J. GENET. & BREED., Bd. 46, - 1992 Seiten 391-393, XP000677745 ROHDE W. ET AL.,: "An EcoRI repetitive sequence family of the coconut palm Cocos nucifera L. shows sequence homology to copia-like elemets" in der Anmeldung erwähnt
- NUCLEIC ACIDS RESEARCH, Bd. 18, Nr. 24, 25.Dezember 1990, Seiten 7213-7218, XP000310554 WELSH J ET AL: "FINGERPRINTING GENOMES USING PCR WITH ARBITRARY PRIMERS"

### SEQUENZPROTOKOLL

(1) ALLGEMEINE ANGABEN:
   (i) ANMELDER:
      (A) NAME: Max-Planck-Gesellschaft zur Förderung der Wissenschaften E.V.
      (B) STRASSE:
      (C) ORT: Berlin
      (D) BUNDESLAND:
      (E) LAND: BRD
      (F) POSTLEITZAHL:
   (ii) BEZEICHNUNG DER ERFINDUNG: Verwendung von Primern für universelle Fingerprint-Analysen
   (iii) ANZAHL DER SEQUENZEN: 13
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.30
   (EPA)
(2) ANGABEN ZU SEQ ID NO: 1:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotid"
   (iii) HYPOTHETISCH: JA
   (xi) SEQUENZBESCHREIBUNG: SEQ ID NO: 1:
   AGGAGGTGAA TACCTTAG
   18
(2) ANGABEN ZU SEQ ID NO: 2:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotid"
   (iii) HYPOTHETISCH: JA
   (xi) SEQUENZBESCHREIBUNG: SEQ ID NO: 2:
   AAAATGGCAT AGTCTCTC
   18
(2) ANGABEN ZU SEQ ID NO: 3:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotid"
   (iii) HYPOTHETISCH: JA
   (xi) SEQUENZBESCHREIBUNG: SEQ ID NO: 3:
   GTCGACATGC CATCTTTC
   18
(2) ANGABEN ZU SEQ ID NO: 4:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotid"
   (iii) HYPOTHETISCH: JA
   (xi) SEQUENZBESCHREIBUNG: SEQ ID NO: 4:
   TATAGTACCT ATTGGGTG
   18
(2) ANGABEN ZU SEQ ID NO: 5:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotid"
   (iii) HYPOTHETISCH: JA
   (xi) SEQUENZBESCHREIBUNG: SEQ ID NO: 5:
   ATATATGGAC TTAAGCAAGC
   20
(2) ANGABEN ZU SEQ ID NO: 6:
   (i) SEQUENZKENNZEICHEN:
      (A) LÄNGE: 21 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotid"
   (iii) HYPOTHETISCH: JA
   (xi) SEQUENZBESCHREIBUNG: SEQ ID NO: 6:
   GTATTGTACG TGGATGACAT C
   21
(2) ANGABEN ZU SEQ ID NO: 7:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotid"
   (iii) HYPOTHETISCH: JA
   (xi) SEQUENZBESCHREIBUNG: SEQ ID NO: 7:
   CAACAGTGCT CCCACTGA
   18
(2) ANGABEN ZU SEQ ID NO: 8:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotid"
   (iii) HYPOTHETISCH: JA
   (xi) SEQUENZBESCHREIBUNG: SEQ ID NO: 8:
   TGCTAGGACT TTCACAGA
   18
(2) ANGABEN ZU SEQ ID NO: 9:
   (i) SEQUENZKENNZEICHEN:
      (A) LÄNGE: 19 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotid"
   (iii) HYPOTHETISCH: JA
   (xi) SEQUENZBESCHREIBUNG: SEQ ID NO: 9:
   TTTTCTACTT CATGTCTGA
   19
(2) ANGABEN ZU SEQ ID NO: 10:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotid"
   (iii) HYPOTHETISCH: JA
   (xi) SEQUENZBESCHREIBUNG: SEQ ID NO: 10:
   AATAAATCGA TCATCGAC
   18
(2) ANGABEN ZU SEQ ID NO: 11:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotid"
   (iii) HYPOTHETISCH: JA
   (xi) SEQUENZBESCHREIBUNG: SEQ ID NO: 11:
   ATTCCCATCT GCACCAAT
   18
(2) ANGABEN ZU SEQ ID NO: 12:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotid"
      (iii) HYPOTHETISCH: JA
      (xi) SEQUENZBESCHREIBUNG: SEQ ID NO: 12:
   ATGTCATCCA CGTACAAT
   18
(2) ANGABEN ZU SEQ ID NO: 13:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotid"
   (iii) HYPOTHETISCH: JA
   (xi) SEQUENZBESCHREIBUNG: SEQ ID NO: 13:
   CTTCTGTGAA AGTCCTAG
   18

## Beschreibung

Die Erfindung betrifft die Verwendung von Primern oder Primerpaaren zur DNA-Fingerprint-Analyse, wobei mit den Primern oder Primerpaaren Fingerprints sowohl vom Menschen, als auch von Tieren, als auch von Pflanzen, als auch von Mikroorganismen erhältlich sind. Die Erfindung betrifft ferner Primer oder Primerpaare zur vorstehend genannten Verwendung.

Es ist allgemein bekannt, daß die Anwesenheit polymorpher und heterogen verteilter repetitiver Sequenzen wie Mikrosatelliten für genetische Analysen Verwendung finden.

Es ist auch allgemein bekannt, daß Retrotransposons wie die copia-Elemente aus Drosophila und copia-ähnliche Elemente in anderen Spezies des Tier- und Pflanzenreichs in der Regel als Mehrfachkopien in Genomen enthalten sind. Repetitive Genomsequenzen dieser Art sind am Beispiel copia-ähnlicher Elemente in Pisum (Erbse) zur genetischen Analyse dieser Pflanzenspezies benutzt worden (Lee u.a., Plant Mol. Biol. 15: 707-722, 1990). Diese von den Autoren als OFLP bezeichnete Methode basiert auf einem copia-spezifischen Primer und als zweitem Primer für die PCR-Amplifikation einer Sequenz aus dem diese Retrotransposons flankierenden Erbsengenom. Damit ist es möglich geworden, Erbsensorten durch PCR-Amplifikation bestimmter Elemente der Erbsen-copia-Familie zu amplifizieren und durch Auftrennung der nicht-radioaktiv markierten PCR-Produkte im Agarosegel auf Polymorphismen zu testen und genetische Verwandtschaften zu bestimmen. Auch andere Retrotransposons, z.B. Tos1-1, Tos2-1 und Tos3-1 aus Reis haben als molekulare genetische Marker zur Differenzierung und Identifizierung von Reis-Kultivaren durch RFLP-Analyse Verwendung gefunden (Fukuchi u.a., Jap. J. Genetics, 68: 195-204, 1993), wobei aber auch hier postuliert wurde, daß für andere Pflanzenspezies deren endogene Retrotransposons als molekulare Marker isoliert werden. Eine andere Arbeit (Purugganan und Wessler, Mol. Ecology 4: 265-269, 1995) benutzt eine auf PCR-basierende Methode, welche die Variation in Spaltstellen für Restriktionsenzyme auf transponierbaren Elementen für eine Fingerprint-Analyse ausnutzt. Allen diesen im Stand der Technik beschriebenen Verfahren ist jedoch gemeinsam, daß die dort beschriebenen genetischen Marker bzw. Primer nicht universell bei Menschen, Pflanzen, Tieren oder Mikroorganismen einsetzbar sind. Es liegt auf der Hand, daß die Bereitstellung derartiger genetischer Marker oder Primer in vielen Bereichen der modernen Biologie oder Medizin wesentliche Vorteile mit sich bringen würde.

Aufgabe der vorliegenden Erfindung war daher, die vorstehend beschriebenen Nachteile aus dem Stand der Technik zu überwinden und Wege und Mittel bereitzustellen, die eine möglichst universelle Anwendbarkeit einer möglichst geringen Anzahl von Primern bzw. genetischen Markern bei der Fingerprint-Analyse von Arten sowohl aus dem Tier- als auch aus dem Pflanzenreich wie auch beim Menschen gestattet.

Die Lösung dieser Aufgabe wird durch die in den Ansprüchen gekennzeichneten Ausführungsformen erreicht.

Überraschenderweise wurde nämlich nunmehr gefunden, daß Primer, die mit dem copia-ähnlichen Element aus der Kokosnuß (Cocos nucifera L.) hybridisieren, und dort eine Fingerprint-Analyse ermöglichen, auch bei vielen anderen Spezies aus dem Tier- und Pflanzenreich wie auch beim Menschen und sogar Mikroorganismen einschließlich der Hefe mit Erfolg eingesetzt werden können. Dieser Befund erlaubt die universelle Anwendbarkeit der genannten Primer zur Fingerprint-Analyse im gesamten Tier- und Pflanzenreich sowie beim Menschen und bei Mikroorganismen.

Die Erfindung betrifft somit die Verwendung eines Primers oder Primerpaares wobei der Primer oder das Primerpaar einer DNA-Sequenz der DNA-Fragmente der copia-ähnlichen Elemente entspricht, die in der nach Restriktion isolierter genomischer DNA aus Kokosnuß (Cocos nucifera L.) mit dem Restriktionsenzym EcoRI und Auftrennung im Agarosegel als zwei Fragmente, jeweils mit 1.3 und 1.4 Kilobasen, sichtbar sind, zur DNA-Fingerprint-Analyse von Menschen, Tieren, Pflanzen oder Mikroorganismen, und wobei
(a) das Tier aus dem Tierreich mit allen Unterreichen, vorzugsweise die Metazoen, darin, enthalten die Unterstämme der Vertebraten, dabei vorzugsweise die Klasse der Säugetiere, darin enthalten insbesondere die Familie der Hominiden und die Familie der Bovidae, darin enthalten die Spezies Bovis tourus und Ovis aries, sowie alle Rassen und Varietäten, die sich aus der entsprechenden Spezies ableiten lassen, stammt;
(b) die Pflanze aus dem Unterreich der Mycobionta, aus der Familie der Poaceae mit ihren Vertretern der Spezies Hordeum vulgare und Zea mays, oder aus der Klasse der Dicotyledonae mit ihren Familien, z.B. der Solaneceae und ihrem Vertreter der Spezies Solanum tuberosum, Nicotiana tabacum, Petunia hybrida, oder z.B. die Familie der Brassicaceae mit ihrem Vertreter der Spezies Brassica napus oder die Familie der Chenopodiaceae mit ihrem Vertreter Beta vulgaris oder die Familie der Vitaceae mit ihrem Vertreter Vitis vinifera sowie alle Varietäten und Sorten, die sich von der entsprechenden Spezies ableiten lassen, stammt;
(c) Mikroorganismen, prokaryotische Mikroorganismen, dabei vorzugsweise Gram-positive Bakterien wie z.B. Milchsäurebakterien, Sarcina und Coryneforme Bakterien und Gram-negative Bakterien wie z.B. Neisseria und Enterobakterien, und eukaryotische Mikroorganismen umfassend Pilze, dabei vorzugsweise Phycomyceten wie z.B. Phytophthora und Ascomyceten wie z.B. Hefen umfassen.

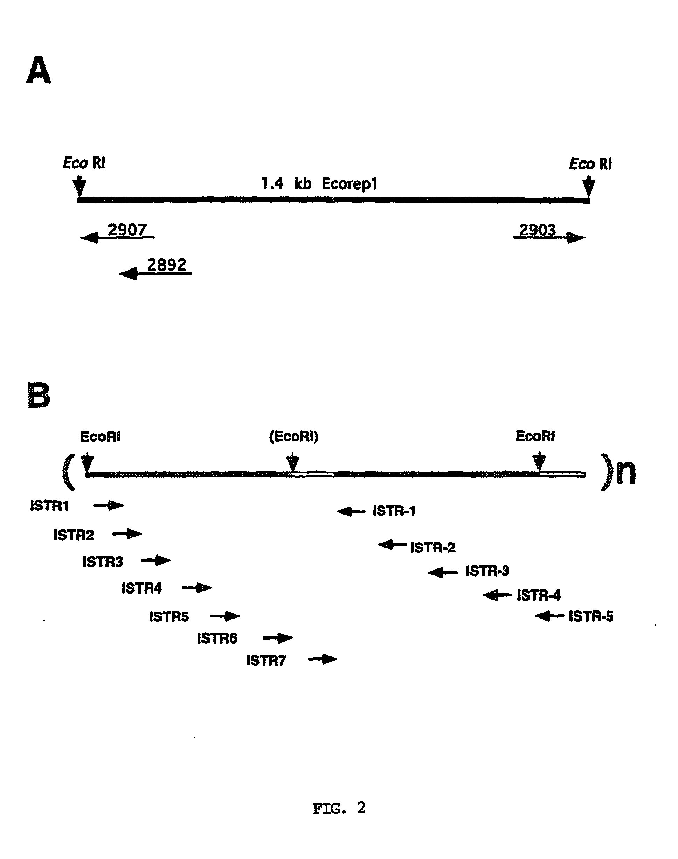

Dabei werden die in dieser Erfindung beschriebenen überraschenden Ergebnisse sowohl mit beliebigen Kombinationen unterschiedlicher Primer gegenläufiger Orientierung erreicht, die nur die Bedingung erfüllen müssen, daß sie an das oben genannte, in Figur 2B dargestellte copia-ähnliche Element hybridisieren, als auch unter Einsatz eines einzigen Primers, der aufgrund der Repetition des copia-ähnlichen Elements, allerdings in 5'→3'/3'→5' Orientierung zweier benachbarter Elemente und nicht wie in Figur 2B dargestellt, in 5'→3'/5'→3'-Orientierung, ebenfalls die hochpolymorphen Fingerprints bereitstellt. Die vorstehend gewählte Begriffsbestimmung für die Primer schließt selbstverständlich ein, daß diese auch an DNAs anderer Organismen hybridisieren, sofern diese DNA-Sequenzen enthalten, die DNA-Sequenzen aus dem vorstehend genannten copia-ähnlichen Element entsprechen.
Die Bedingungen, unter denen eine Hybridisierung der Primer und nachfolgende Amplifikation erfolgt, ist für den Fachmann ohne erfinderisches Bemühen aus dem Stand der Technik und dem nachfolgenden Beispielen ableitbar.

Die Länge der in dieser Erfindung verwendeten Primer beträgt vorzugsweise 15 bis 25 Nucleotide. Allerdings ist die Erfindung auch mit kürzeren oder mit längeren Primern durchführbar.

Der vorliegende Befund ist umso überraschender, als in der Regel im Stand der Technik davon ausgegangen worden ist, daß Primer lediglich in taxonomisch eng gesteckten Grenzen eingesetzt werden können, wenn aussagekräftige Fingerprints erhalten werden sollen.

Im Stand der Technik wurde von Rohde u.a. (J. Genet. & Breed., 46: 391-394, 1992) beschrieben, daß im Genom der Kokosnuß (Cocos nucifera L.) hochrepetitive Sequenzen mit Homologie zu auch in anderen Spezies beschriebenen copia-Elementen vorhanden sind, die nach Restriktion isolierter genomischer DNA mit dem Restriktionsenzym EcoRI und Auftrennung im Agarosegel als zwei, jeweils 1.3 und 1.4 Kilobasen große DNA-Banden sichtbar sind. Drei dieser "Ecorep" genannten DNA-Fragmente wurden nach Subklonierung sequenziert, und es konnten Sequenzunterschiede festgestellt werden. Versuche, diese Unterschiede für die genetische Analyse verschiedener Kokosnuß-Typen durch die Verwendung von Ecorep-Sequenzen als molekulare Sonde in RFLP-Analysen oder durch sequenzspezifische PCR-Primer auszunutzen, waren nicht erfolgreich (Rohde u.a., J. Genet. & Breed., 46: 391-394, 1992; Rohde, in: "La Recherche Europeene au Service du Cocotier - Actes du Seminaire - 8-10 septembre 1993, Montpellier". CIRAD (Collection: Colloques du CIRAD), Montpellier, S. 41-52).

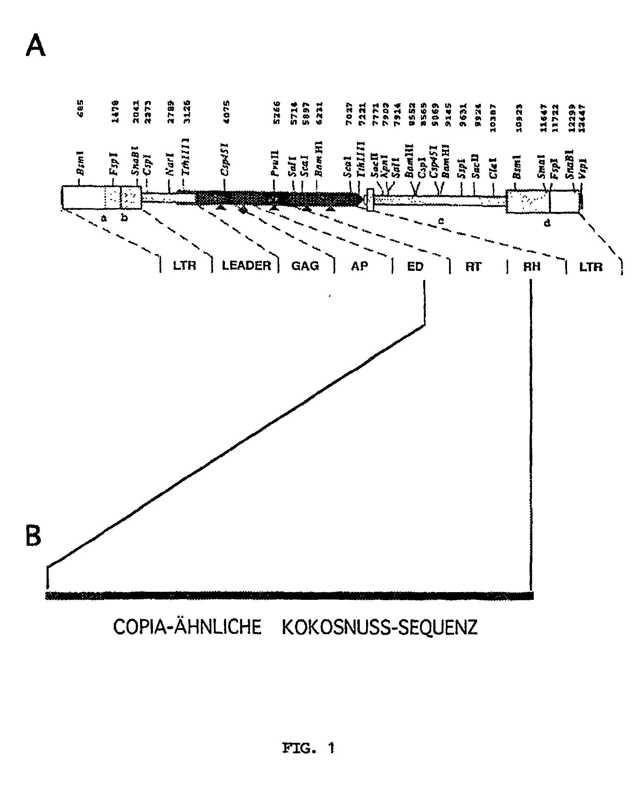

Es wurde kürzlich für drei Kokosnuß-Typen gefunden, daß Subfamilien dieser 1.3 bzw. 1.4 Kilobasen großen Ecorep-Sequenzen existieren, in denen diese Elemente auf dem Kokosnuß-Genom nahe beieinander liegen d.h. in tandem wiederholt sind, und in denen in der Regel zumindest eine der beiden von den früher identifizierten Elementen (Rohde u.a., J. Genet. & Breed., 46: 391-394, 1992) zu erwartenden EcoRI-Spaltstellen an den Enden der zunächst als "Spacer-Region" bezeichneten Sequenz fehlt (Rohde u.a., J. Genet. & Breed., 49: 179-186, 1995). Diese Spacer-Region zeigt hohe Homologie zu dem copia-ähnlichen BARE-1-Element aus Gerste (Fig. 1A; Manninen und Schulman, Plant. Mol. Biol., 22: 829-846, 1993). Bei dieser Subfamilie copia-ähnlicher Sequenzen im Kokosnuß-Genom handelt es sich daher um in tandem wiederholte Sequenzen, die Homologie zur Endonuclease- und Reversen Transkriptase/RNAseH-Region eines copia- bzw. copia-ähnlichen Elements aufweisen (siehe Fig. 1B). Die beobachteten Sequenzunterschiede in den Elementen dieser Subfamilie ließen sich jetzt - im Gegensatz zu den oben beschriebenen Versuchen für die Ecorep-Sequenzen - mit geeigneten PCR-Primern (siehe Fig. 2B) für die genetische Analyse in Kokosnuß ausnutzen. Dieses Verfahren zur Genomanalyse in Kokosnuß wurde als ISTR(inverse sequence-tagged repeat)-Analyse bezeichnet.

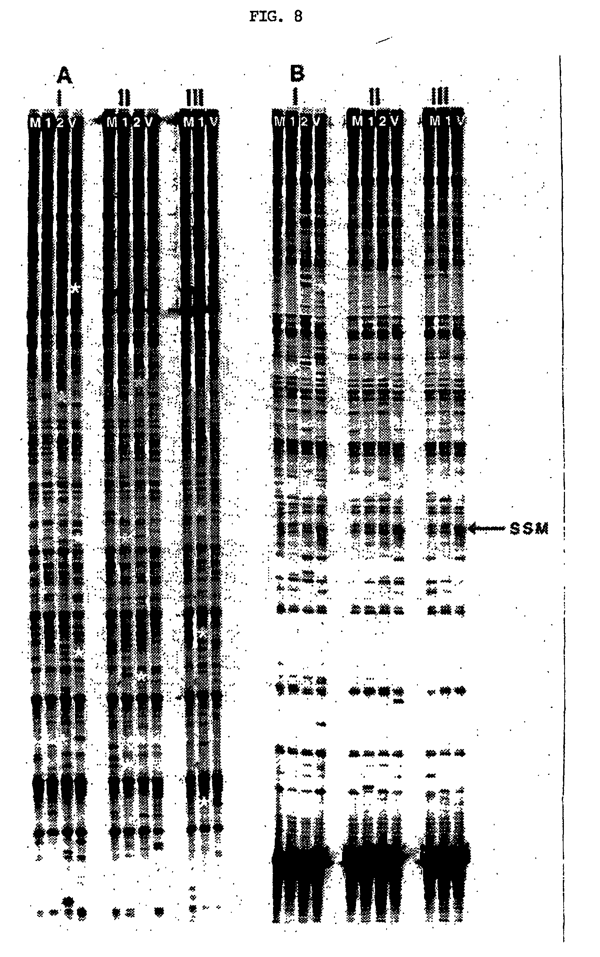

Überraschenderweise wurde nun gefunden, daß diese Subfamilie mit hoher Sequenzkonservierung offensichtlich ubiquitär in der Pflanzen- und Tierwelt vertreten ist, da die Verwendung der identischen ISTR-Primer (siehe auch Tabelle 1), wie sie auf der Basis der von uns ermittelten Kokosnußsequenzen entwickelt wurden, sowohl für andere Pflanzenspezies als auch für Tiere und den Menschen sowie den Mikroorganismen hochpolymorphe DNA-Fingerprints ergibt. Dabei lassen sich nicht nur eine Vielzahl von polymorphen Markern entdecken, die in der Nachkommenschaft segregieren ("single locus/multiple allele"-Marker), sondern es entstehen auch neue polymorphe Marker (individuum-spezifische Marker), die z.B. in kontrollierten Kreuzungen (Beispiele Rind, Schaf) weder im Vater noch in der Mutter vorhanden sind und möglicherweise auf Rekombinationsereignisse oder die Amplifikation bestimmter genomischer Bereiche zurückzuführen sind. Jeder Fingerprint ist folglich einzigartig für den individuellen Nachkommen bei identischen Eltern. Im Humanbereich konnte gezeigt werden, daß dies sogar für eineiige Zwillinge gilt, die für mehrere der verwendeten ISTR-Primer-Paare voneinander verschiedene Fingerprints zeigten (siehe Fig. 8).

Besonders vorteilhaft im Sinne der erfindungsgemäßen Verwendung ist, daß Fingerprints vergleichbarer Auflösung und Sensitivität mit DIG-markierten PCR-Produkten direkt im Gel ohne die generell in bekannter Weise vorgenommene Übertragung der DNA-Fragmente auf Membranen (Southern blot) sichtbar gemacht wurden. Damit ist die Erstellung derartiger Fingerprints in einfachster Weise (Auftrennung der PCR-Fragmente im Sequenzgele, direkter Nachweis im Gel, computer-unterstützte Datenanalyse durch direktes Einscannen der Sequenzgele) ohne die Verwendung von Radioaktivität ermöglicht worden.

Somit ist eine weitere bevorzugte Ausführungsform der erfindungsgemäßen Verwendung dadurch gekennzeichnet, daß die zu analysierenden DNAs mit dem Primer oder dem Primerpaar durch PCR amplifiziert und nachfolgend der Größe nach auf einem Gel aufgetrennt werden.
Der Fachmann weiß aus dem Stand der Technik, wie er die Bedingungen für eine geeignete PCR auszuwählen hat. Auch Verfahren zur Auftrennung von PCRamplifizierten DNAs auf einem Elektrophorese-Gel, das vorzugsweise ein Polyacrylamidgel ist, ist dem Stand der Technik zu entnehmen.

In einer besonders bevorzugten Ausführungsform ist das Gel ein Sequenzgel. Die Herstellung von Sequenzgelen ist ebenfalls im Stand der Technik bekannt und beispielsweise beschrieben in Sambrook et al., "Molecular Cloning, A Laboratory Handbook", CSH Press, Cold Spring Harbor, 1989.

In einer weiteren bevorzugten Ausführungsform ist die erfindungsgemäße Verwendung dadurch gekennzeichnet, daß als weiterer Schritt ein Southern Blot durchgeführt wird, und die auf die Membran übertragenen DNAs durch Hybridisierung mit einer Sonde sichtbar gemacht werden.
Diese Ausführungsform ist als Alternative zu den vorstehend beschriebenen beiden Ausführungsformen zu sehen. Sie erfordert zwar mehr Aufwand und den Umgang mit Radioaktivität, ist jedoch durchaus für Labors geeignet, die eine weniger aufwendige Laboreinrichtung betreiben, so z.B. keinen Scanner mit daran angeschlossenen Computer besitzen. Die Durchführung von Southern Blots sowie die Hybridisierungen mit einer geeigneten Sonde sind ebenfalls im Stand der Technik bekannt und beispielsweise in Sambrook et al., a.a.O., beschrieben.

In einer weiteren besonders bevorzugten Ausführungsform der erfindungsgemäßen Verwendung ist die Sonde der erfindungsgemäße Primer bzw. das erfindungsgemäße Primerpaar.
Da die Primer Bestandteil der amplifizierten DNA sind, ist durch sie in einfacher Weise auch ein Nachweis der Banden auf der für den Southern Blot verwendeten Membran möglich.

In einer weiteren bevorzugten Ausführungsform der erfindungsgemäßen Verwendung trägt der Primer oder das Primerpaar eine Markierung.

In einer besonders bevorzugten Verwendung ist die Markierung eine nicht-radioaktive Markierung, insbesondere Digoxigenin, Biotin und Fluoreszenzfarbstoff, ein Farbstoff oder eine radioaktive Markierung, insbesondere ³²P.
Insbesondere die Markierung der Primer mit Digoxigenin und die Anfärbung nach Amplifikation der DNA und gelelektrophoretischer Auftrennung direkt im Gel kann von allen Labors oder interessierten Züchtern unter Einsatz eines geringen Geräteaufwands (PCR-Reaktion, Elektrophorese auf Sequenzgelen) und Verzicht auf Radioaktivität verwendet werden können. Die Speicherung und Verarbeitung der Daten geschieht vorzugsweise durch direktes Einlesen des gefärbten und getrockneten Gels mittels Scanner in einen Computer. Weiterhin ist die Möglichkeit gegeben, durch Reisolierung von PCR-Produkten aus dem Sequenz-Gel, Reamplifikation und Sequenzierung spezifische Primer zu entwickeln, die allel-spezifische Amplifizierungsprodukte ergeben.

In einer weiteren bevorzugten Ausführungsform der Erfindung weist der Primer die in Tabelle 1 angegebene Sequenz auf.
Diese Primer sind bevorzugte Beispiele der von den Erfindern in bisherigen DNA-Fingerprint-Analysen angewendeten Primer. Es ist jedoch hervorzuheben, daß auch andere Primer die an die in Figur 2B schematisch dargestellte Sequenz hybridisieren, die in Rohde et al., 1992, a.a.O., und Rohde et al., 1995, a.a.O., näher beschrieben ist, verwendet werden können. Erfindungsgemäß hat sich nämlich ferner überraschenderweise herausgestellt, daß sämtliche bisher getesteten Primer, die an diesem Bereich hybridisieren, einen aussagekräftigen Fingerprint sowohl bei Pflanzen, als auch bei Tieren, als auch bei Menschen, als auch bei Mikroorganismen ergeben können.

In einer weiteren bevorzugten Ausführungsform ist die erfindungsgemäße Verwendung dadurch gekennzeichnet, daß die Fingerprint-Analyse für Biodiversitätsstudien, Studien zur genetischen Verwandtschaft, taxonomische Studien, und insbesondere in der Rechtsmedizin, der Züchtung, im Sortenschutz, im Genbank-Management, in der Populationsgenetik und für Evolutionsstudien eingesetzt wird.

Schließlich betrifft die Erfindung Primer zur erfindungsgemäßen Verwendung, die dadurch gekennzeichnet sind, daß die Primer eine der in Tabelle 1 dargestellten Sequenzen aufweisen.

Die Figuren zeigen:
- **Fig.1:**: Bereich eines im Gerstengenom vorkommenden copia-ähnlichen Elements Bare-1 (Fig. 1A, aus Manninen und Schulman, Plant. Mol. Biol., 22: 829-846, 1993), der als in tandem wiederholte copia-ähnliche Sequenz (Rohde u.a., J. Genet. & Breed., 49: 179-186, 1995) im Genom der Kokosnuß (Cocos nucifera L.) gefunden wurde (Fig. 1B).
(A) Schematische Darstellung des copia-ähnlichen BARE-1-Elements aus Gerste.
   ED: Endonuklease; RT: Reverse Transkriptase; RH: RNAse H.
(B) Lage von repetitiven copia-ähnlichen Sequenzen aus der Kokosnuß relativ zu homologen Sequenzen auf dem Gersten-BARE-1-Element. Der schraffierte Bereich kennzeichnet die Position der kürzlich gefundenen "Spacer-Region" (Rohde u.a., J. Genet. & Breed., 49: 179-186, 1995).
- **Fig. 2:**: Amplifikation der "Spacer-Region" zwischen benachbarten copia-ähnlichen Sequenzen im Kokosnuß-Genom (A) und ungefähre Position bisher verwendeter Primer für die ISTR-Analyse (B).
(A) Für die Amplifikation zur Klonierung und Sequenzierung der Regionen zwischen zwei benachbarten copia-ähnlichen Elementen der Kokosnuß wurden die Primerpaare ISTR5/ISTR-1 und ISTR5/ISTR-2 verwendet. Die Richtung der Pfeile symbolisiert die 5'→3'-Orientierung der verwendeten Oligodeoxynukleotide.
(B) Die einzelnen Primer sind in der Regel zwischen 18 und 20 Nukleotiden lang und wurden analog zur Sequenz des Ecorep1-Elements synthetisiert (Rohde u.a., J. Genet. & Breed., 49: 179-186, 1995). Die mit "-" versehenen Primer sind komplementär zur kodierenden Sequenz des copia-Elementes und können mit jedem beliebigen Primer der "plus"-Serie für die ISTR-Analyse kombiniert werden.

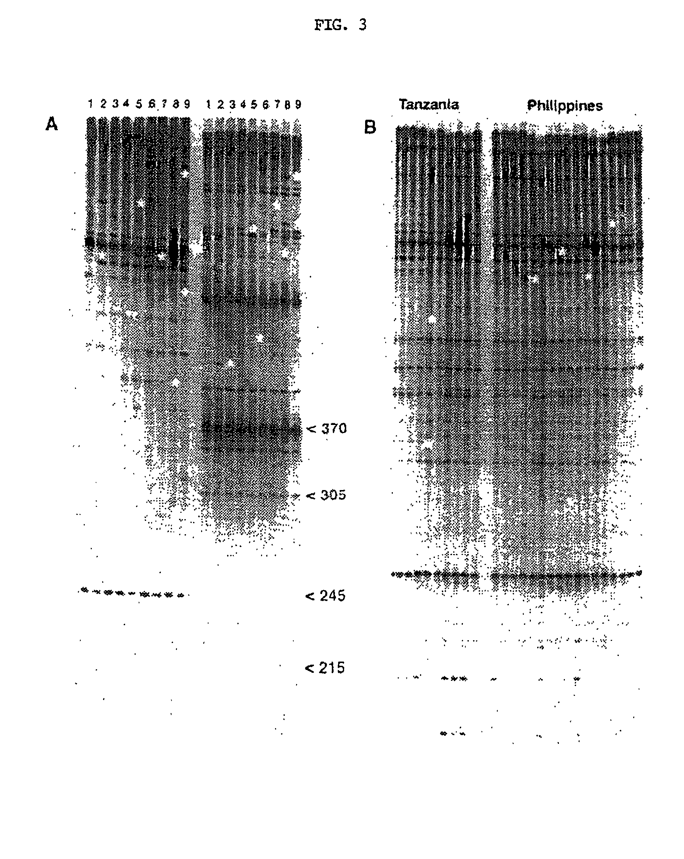

- **Fig. 3:**: ISTR-Analyse von Populationen am Beispiel der Kokosnuß (aus Rohde u.a., J. Genet. & Breed., 49: 179-186, 1995).
(A) In den Spuren 1 bis 7 wurden einzelne Palmen einer East African Tall (EAT) Population durch ISTR-Analyse mit den Primerpaaren ISTR5/ISTR-2 (links) bzw. ISTRS/ISTR-1 (rechts) charakterisiert. In den Spuren 8 und 9 sind Kontrollanalysen einer einzelnen Rennell Island Tall(RLT)- oder Pemba Red Dwarf(PRD)-Palme aufgetragen.
(B) ISTR-Analyse zweier Malayan Yellow Dwarf(MYD)-Populationen aus Tanzania und den Philippinen mit dem Primerpaar ISTR5/ISTR-2.
- **Fig. 4:**: Generelle Anwendung von ISTR-Primern im Pflanzenbereich. DNA verschiedener Pflanzenspezies wurde einer Amplifikation mit den Primern ISTR5/ISTR-2 unterzogen. In den einzelnen Spuren wurden die PCR-Produkte folgender Pflanzen aufgetragen:
1: Tabak, 2: Gerste, 3: Kartoffel, 4: Mais, 5: Antirrhinum, 6: Arabidopsis, 7: Raps, 8: Craterostigma, 9: Petunie, 10: Petersilie, 11: Sisal, 12: Milala-Palme, 13: Borassus-Palme, 14: Kokospalme, 15: Zuckerrube, 16: Cuphea, 17: Hefe.

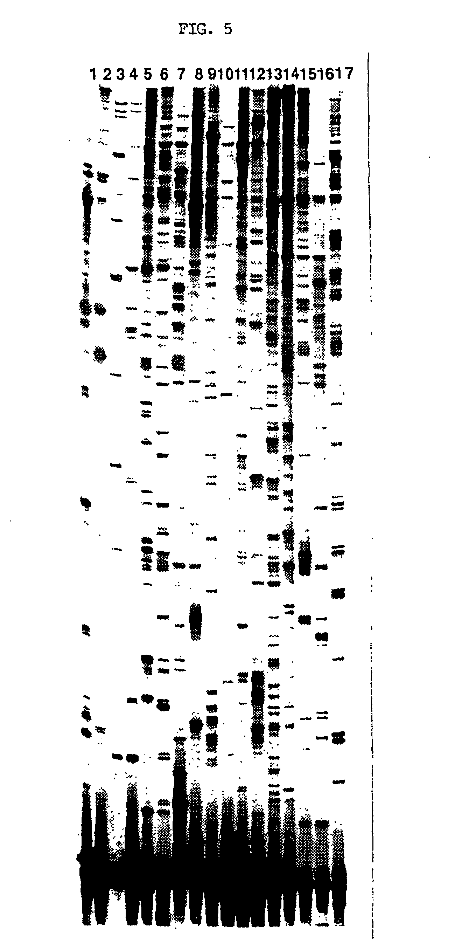

- **Fig. 5:**: ISTR-Analyse von einzelnen Angehörigen der Familie der Arecaceae (Palmae). DNAs von 17 verschiedenen Palmenarten wurden in einer Standard-PCR-Reaktion mit den Primern ISTR5/ISTR-2 amplifiziert und auf einem 4% PAGE-Gel aufgetrennt. In den einzelnen Spuren sind die PCR-Produkte folgender Pflanzen aufgetragen: 1: Hyphaene petersiana Mart.; 2: Bismarckia nobilis Hildebrandt & H. Wendl.; 3: Eugeissona utilis Becc.; 4: Korthalsia echinometra Becc.; 5: Mauritiella aculeata (H.B. & K.) Burret; 6: Nypa fruticans Wurmb.; 7: Pseudophoenix sargentii H. Wendl. ex Sarg.; 8: Oraniopsis appendiculata (F.M.Bailey) J.Dransf., Irvine and N.W.Uhl; 9: Socratea exorhizza (Mart.) H.Wendl.; 10: Halmoorea tripatha J. Dransf. & N.W.Uhl.; 11: Cyrtostachys peekeliana Becc.; 12: Deckenia nobilis H.Wendl.; 13: Oncosperma tigillarium (Jack) Ridley; 14: Syagrus amara (Jacq.f.) Mart.; 15: Attalea allenii H.E.Moore ex L.H.Bailey; 16: Scheelea insignis (Mart.) Karsten; 17: Asterogyne martiana (H.Wendl.) H.Wendl. ex Hemsley.
- **Fig. 6:**: ISTR-Analyse von Gerstensorten.
DNAs von 35 verschiedenen Gersten-Genotypen wurden in einer Standard-PCR-Reaktion mit den Primern ISTR5/ISTR-2 amplifiziert und auf einem 4% PAGE-Gel aufgetrennt. In den einzelnen Spuren sind die PCR-Produkte folgender Pflanzen aufgetragen: 1: Fiction; 2: Kaskade; 3: Red; 4: Georgie; 5: Alexis; 6: Marinka; 7: Flash; 8: Portikos; 9: Aura; 10: Gimpel; 11: Prisma; 12: Gitane; 13: Gavotte; 14: Manila; 15: Pilastro; 16: Masto; 17: Torrent; 17: Torrent; 18: Thibault; 19: Onice; 20: Mette; 21: Robur; 22: Probidor; 23: Tania; 24: Mario Otter; 25: Nico; 26: Magie; 27: Vogelsanger Gold; 28: Tekto 2002; 29: Asse; 30: Calcaroides-C15 (ex Bonus); 31: calcaroides-b2 (ex Bonus); 32: calcaroides-b19 (ex Bonus); 33: Bonus; 34: Christina; 35: Nudinka.

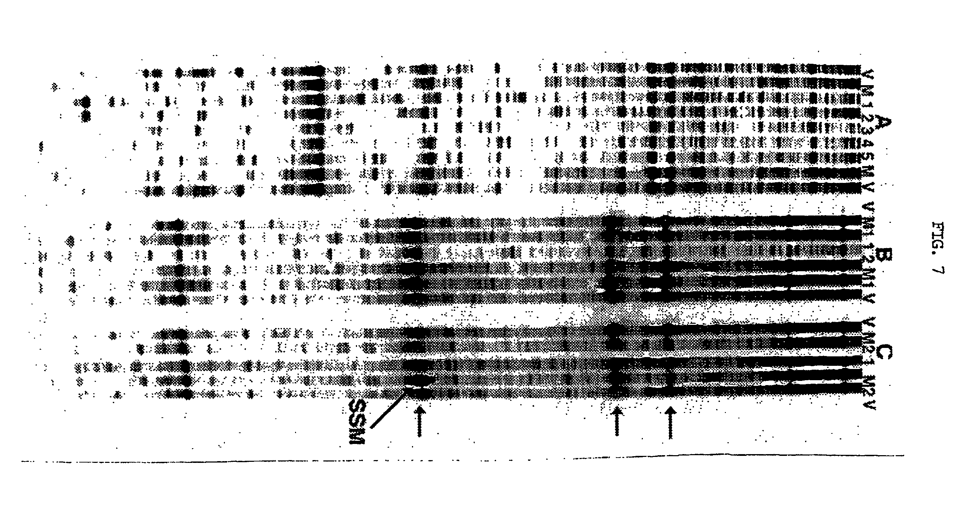

- **Fig. 7:**: Analyse einer Rinderfamilie (A) und zweier Schafsfamilien (B, C).
(A) Fünf Nachkommen sowie die beiden Eltern einer Rinderfamilie wurden einer ISTR-Analyse mit dem Primerpaar ISTR5/ISTR-2 unterzogen. V: Vater; M: Mutter. Die einzelnen Nachkommen sind numeriert. Der Pfeil deutet auf einen Marker, der nicht in allen Nachkommen auftritt.
(B, C) Analyse zweier Schafsfamilien mit Nachkommen einer Kreuzung zwischen dem identischen Vater und Mutter M1 (B) sowie Mutter M2 (C). Pfeile zeigen segregierende ISTR-Marker an; Sterne deuten auf individuum-spezifische Marker, die weder in den Eltern noch in den Geschwistern vorhanden sind.
GSM: Marker (untere Bande des Triplets), der mit der männlichen Geschlechtsausprägung kosegregiert. V: Vater. Die einzelnen Nachkommen der verschiedenen Züchtungen sind numeriert.
- **Fig. 8:**: Analyse dreier Menschenfamilien I, II und III mit verschiedenen Primerpaaren.
(A) ISTR-Analyse mit dem Primerpaar ISTR6/ISTR-1. (B) ISTR-Analyse mit dem Primerpaar ISTR6/ISTR-2. V: Kindvater; M: Mutter; SSM: sexspezifischer Marker. Die Nachkommen sind numeriert. Die beiden Nachkommen der Familien I und II sind eineiige Zwillinge.

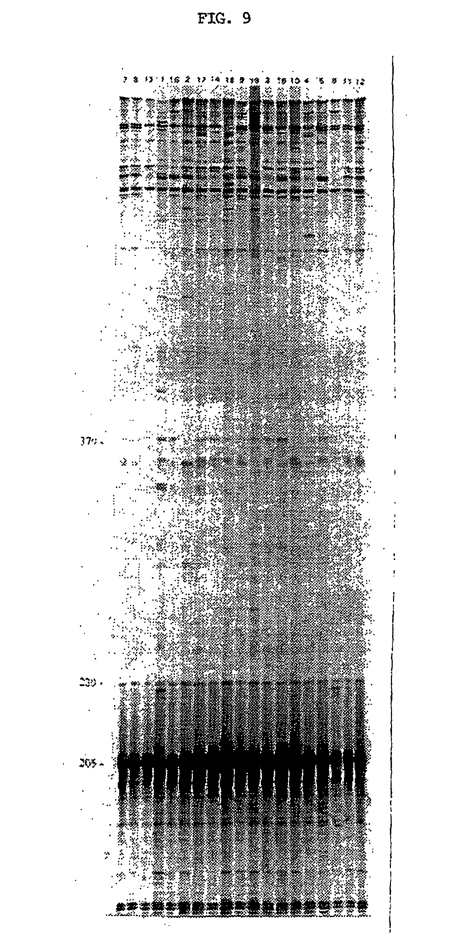

- **Fig. 9:**: Figur 9 zeigt die DNA Analyse von Weinsorten. Für die ISTR-Fingerprint-Analyse wurde DNA aus 19 verschiedenen Weingenotypen mit dem Primerpaar ISTR5/ISTR-2 einer PCR-Reaktion unterzogen. In den einzelnen Spuren wurden die PCR-Produkte folgender Pflanzen aufgetragen:
1. Sangiovese piccolo precoce, 2. Sangiovese dell'Elba, 3. Sangiovese polveroso Bonechi, 4. Colorino americano, 5. Prugnolino medio, 6. Colorino del Valdarno, 7. Morellino, 8. Brunellone, 9. Sangiovese forte, 10. Sangiovese R10, 11. Saragiolo, 12. Colorino di Pisa, 13. Prugnolino dolce, 14. Morellino di Scansano, 15. Colorino di Lucca, 16. Giacchè, 17. Tinturiér, 18. Sangiovese polveroso, 19. Prugnolo gentile.

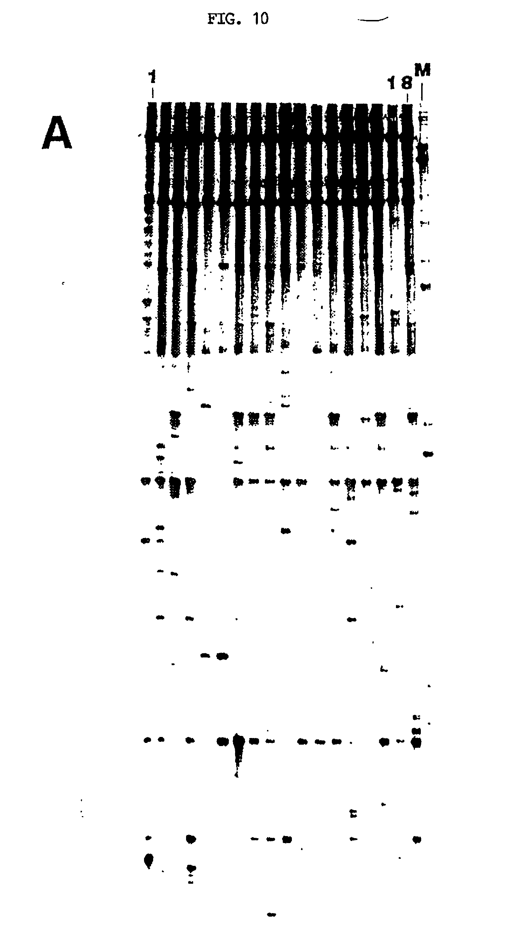

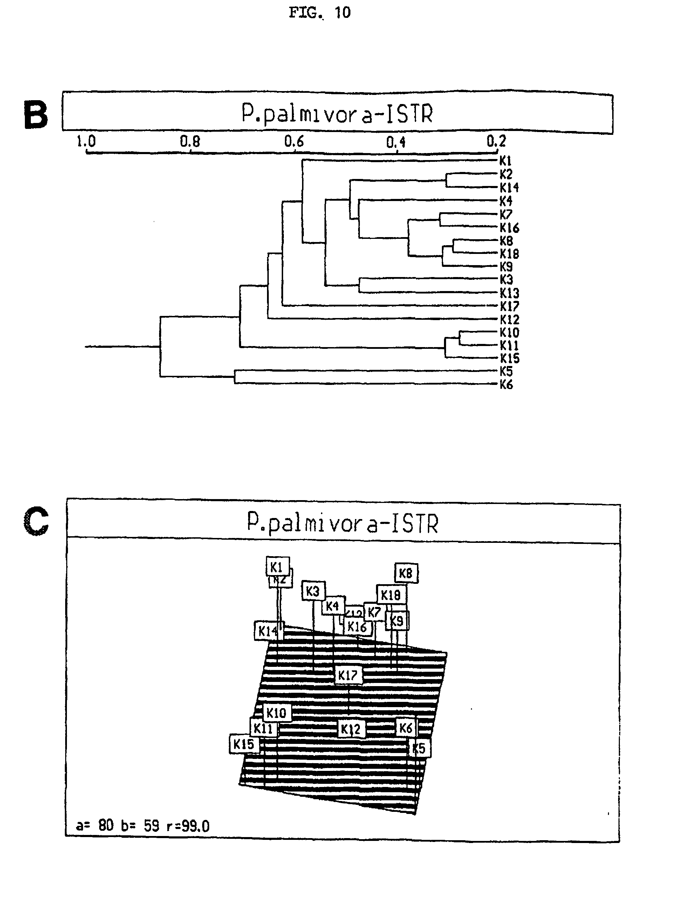

- **Fig. 10:**: Analyse von Phytophthora palmivora-lsolaten aus den Philippinen mit der Primer-Kombination ISTR5/ISTR-2
1: #P8704 (DRC089; Davao City, Mindanao); 2: #P8646 (DRC001; Davao Sur, Mindanao); 3: #P8652(DRC007; Davao City, Mindanao); 4: #P8650 (DRC005; Davao City, Mindanao); 5: #P8698 (DRC082; Zamboanga, Mindanao); 6: #P8684 (DRC065; De Oro City, Mindanao); 7: #P8676 (DRC053; Davao City, Mindanao); 8: #P8653 (DRC008; Davao Norte, Mindanao); 9: #P8647 (DRC002; Davao Norte, Mindanao); 10: #P8649 (DRC004; Davao Norte, Mindanao); 11: #P8662; 12: #P8663 (DRC030; Davao Norte, Mindanao); 13: #P8667 (DRC036; South Cotabato, Mindanao); 14: #P8651 (DRC006; Davao Sur, Mindanao); 15: #P8674 (DRC047; Batangas, Luzon); 16: #P8660 (DRC025; Laguna, Luzon); 17: #P8705 (DRC090; Davao Norte, Mindanao); 18: #P8665 (DRC033; South Cotabato, Mindanao).
M: Kontroll-Reaktion mit DNA der MRD(Malayan Red Dwarf)-Kokospalme.

Die Beispiele erläutern die Erfindung.

### Referenz beispiel 1

### Nachweis von Längenpolymorphismen bei der Kokosnuß

Für diesen Versuch, der in Fig. 3 dargestellt ist, wurden die Primerpaare ISTR5/ISTR-2 und ISTR5/ISTR-1 (siehe Tabelle 1) verwendet. Als zu untersuchende DNAs werden die genomischen DNAs von einzelnen Palmen aus Populationen von East African Tall (EAT) und Malayan Yellow Dwarf (MYD) sowie je einer einzelnen Palme Rennel Island Tall (RLT) und Pemba Red Dwarf (PRD) verwendet. Die betreffenden Oligodeoxynukleotide (Primer) wurden mit Hilfe der Polynukleotidkinase in bekannter Weise an ihrem Ende mit ³²P radioaktiv markiert und in einer PCR-Reaktion eingesetzt. Diese wird standardmäßig in einem Volumen von 20 µl durchgeführt und enthält je 1 pmol der Primer und 25 ng der zu amplifizierenden genomischen DNA in 1x PCR-Reaktionspuffer (z.B. der Firma GIBCO/BRL), 2.5 mM MgCl2, 0.25 mM dNTP (Deoxynukleosid-Triphosphate), und 1 Einheit Taq-DNA-Polymerase. Das Gemisch wird zunächst für 3 Min. bei 95°C denaturiert und danach werden insgesamt 40 Zyklen von 95°C (30 Sekunden, Denaturierung), 45°C (30 Sekunden, Anlagerung) und 72°C (2 Minuten, Synthese) durchgeführt. Die Reaktion wird durch einen Syntheseschritt (72°C für 10 Minuten) beendet, 10 µl Farbstoffgemisch (in Formamid) hinzugefügt und nach erneutem Erhitzen 3 µl davon auf einem 4% Polyacrylamid-Sequenzgel aufgetrennt. Das Gel wird nach Trennen der beiden Glasplatten in bekannter Weise an einer der Sequenz-Glasplatten getrocknet und die aufgetrennten radioaktiv markierten PCR-Produkte durch Belichten eines Röntgenfilms sichtbar gemacht. Dieses experimentelle Protokoll gilt auch für alle anderen ISTR-Primer und für die weiteren Beispiele.

Wie aus Fig. 3 hervorgeht, sind eine Reihe von DNA-Produkten allen Palmen gemein, aber es sind in beiden Populationen auch Unterschiede in einzelnen Palmen zu beobachten. Dies ist weniger überraschend für den "Tall"-Typ EAT (Fig. 3A), da für diese Kokosnuß-Typen Fremdbefruchtung im Feld beobachtet worden ist. Überraschenderweise entdeckt die ISTR-Analyse jedoch auch im allgemeinhin als autogam geltenden "Dwarf'-Palmentyp wie MYD Unterschiede innerhalb der Populationen sowie auch Unterschiede zwischen den Populationen aus Tanzania und den Philippinen (Fig. 3B). Mit bisher verwendeten RFLP-Markern konnten Unterschiede in Dwarf-Populationen nicht nachgewiesen werden. Darüberhinaus ist ersichtlich, daß die Verwendung des Primerpaars ISTR5/ISTR-1 nicht nur - wie von der Lage des ISTR-1-Primers erwartet (Fig. 2B) - etwa 100 bp kleinere PCR-Produkte ergibt, sondern auch neue Polymorphismen verursacht. Die Ursache hierfür kann nur vermutet werden, aber dieser Befund eröffnet die Möglichkeit, basierend auf den ermittelten copia-ähnlichen Sequenzen der Kokosnuß alle denkbaren copia-ähnlichen Sequenzen und Primerkombinationen für die ISTR-Analyse einzusetzen. Dieses einfache Experiment zeigt daher eindrucksvoll, wie bereits mit Hilfe einer einzigen PCR-Amplifikation unter Verwendung des identischen Primerpaares eine reproduzierbare Fingerprint-Analyse einzelner Palmen und Aussagen zur genetischen Homogenität von Populationen ermöglicht werden.

### Beispiel 1

### Test auf generelle Anwendung bei Pflanzen

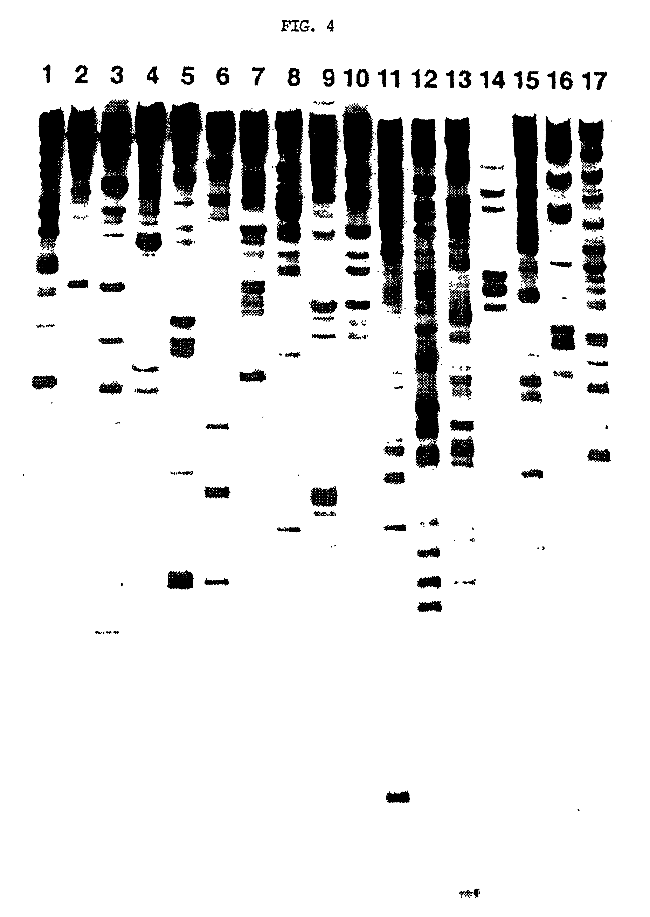

Um die Möglichkeit zu ergründen, die Kokosnuß-spezifischen ISTR-Primer generell in Pflanzen für den Nachweis von DNA-Polymorphismen in copia-ähnlichen Sequenzen anzuwenden, wurden in dem in Beispiel 2 durchgeführten Versuch die genomischen DNAs verschiedener Pflanzen mit dem ISTR-Primerpaar ISTR5/ISTR-2 in einer PCR-Reaktion eingesetzt. Aus Fig. 4 ist ersichtlich, daß von Tabak- bis zu Hefe-DNA alle eingesetzten DNAs durch Kokosnuß-spezifische Primer individuelle PCR-Produkte ergeben. Ähnliche Versuche wurden auch mit anderen ISTR-Primerkombinationen durchgeführt. Dies zeigt, daß ähnliche wie die für Kokosnuß beschriebenen Familien von benachbart gelegenen copia-ähnlichen repetitiven Elementen in niederen und höheren Pflanzen existieren und für eine Fingerprint-Analyse zugänglich sind. Als Konsequenz ist die ISTR-Analyse daher über die in Referenz beispiel 1 für eine einzelne Pflanzenspezies gezeigte Möglichkeit hinaus anwendbar für die Charakterisierung genetischer Diversität und das Erfassen pflanzengenetischer Resourcen, entweder in Genbanken oder durch in situ-Konservierung.

### Referenz beispiel 2

### Test auf Anwendung innerhalb einer Pflanzenfamilie am Beispiel der Palmen (Arecaceae)

Die mögliche Anwendung der ISTR-Analyse zu taxonomischen Studien wurde mit Hilfe des ISTR-Primerpaars ISTR5/ISTR-2 an Pflanzenspezies der Familie Arecaceae (Palmae) durchgeführt. Dazu wurden DNAs von 17 Palmenarten (siehe Legende zu Fig. 5) in einer PCR-Reaktion mit den erwähnten Primern amplifiziert und die PCR-Produkte in bekannter Weise analysiert. Wie aus Fig. 5 ersichtlich, wird für jede Palme ein unterschiedlicher Fingerprint erhalten, der es ermöglicht, die Daten durch Computer-unterstützte Auswertung einer entsprechenden Matrix zur Ermittlung biologischer Diversität durch die Erstellung von Dendrogrammen nach üblichen Verfahren zu verarbeiten. Für die praktische Anwendung ist zum Beispiel von Bedeutung, welche genetische Verwandtschaft etwa zwischen den bedeutenden Ölpflanzen der Öl- und der Kokosnuß-Palme existieren. Genetische Marker etwa für das für die Ölausbeute bedeutsame Merkmal der Nußschalendicke könnten dann in beiden Spezies für die Züchtung Anwendung finden, wenn diese genetisch hochverwandt sind.

### Beispiel 2

### Test auf Anwendung bei gezüchteten Sorten am Beispiel der Gerste

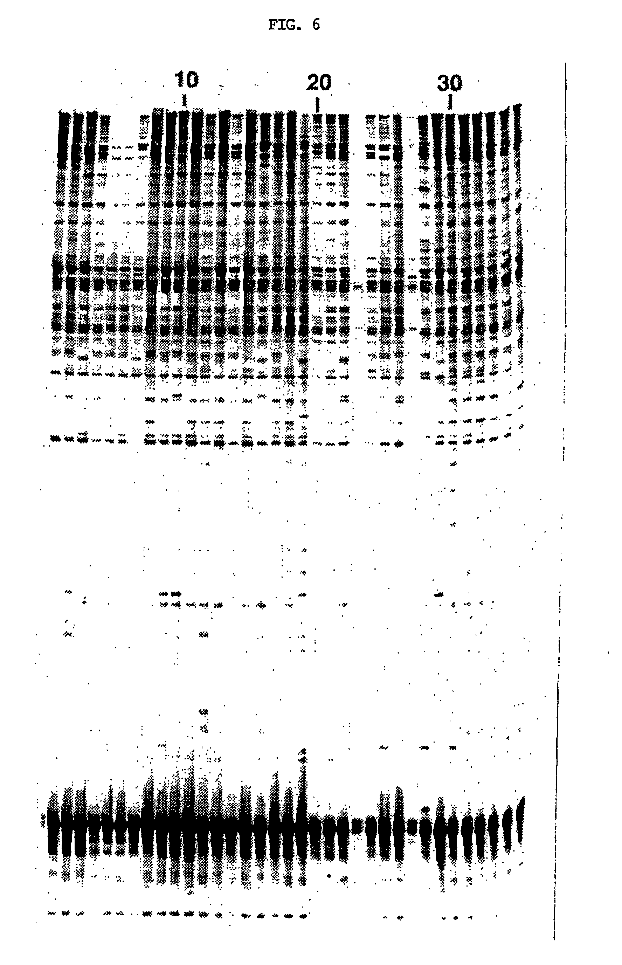

Die Charakterisierung von gezüchteten Sorten durch Fingerprint-Analyse mit Hilfe der ISTR-Technologie wurde am Beispiel von Gerstensorten getestet. Fig. 6 zeigt eine PAGE-Analyse von PCR-Produkten, die für insgesamt 35 Varietäten bzw. Genotypen erhalten wurde. Die hohe genetische Verwandtschaft der untersuchten Hochleistungssorten ist aus der hohen Anzahl von monomorphen DNA-Fragmenten ersichtlich. Dennoch konnten allein aus dieser einen Analyse insgesamt 44 polymorphe Marker identifiziert werden, die vor allem im oberen Bereich des Sequenzgels gelegen waren. Diese Marker wurden in einer Matrix angeordnet und daraus nach der UPGMA-Methode ein Dendrogramm ermittelt. Die Tatsache, daß die Sorte Bonus (Spur 33) nicht von calcaroides-b19 (Spur 32) zu unterschieden ist, ist nicht weiter verwunderlich, da dieser Genotyp eine in Bonus erzeugte rezessive Mutante ist. Dies gilt allerdings auch für die Genotypen Calcaroides-C15 (Spur 30) und calcaroides-b2 (Spur 31), die durch Mutagenese im selben genetischen Hintergrund erzeugt worden waren. Allerdings wurden hier für die Mutagenese Neutronen-(Calcaroides-C15) bzw. Röntgenstrahlen (calcaroides-b2 ) als Mutagene verwendet, die auf chromosomaler Ebene in der Regel zu Deletionen und Inversionen führen, während calcaroides-b19 aus Bonus durch Natriumazid-Behandlung erhalten wurde, die Punktmutationen hervorruft. Dieses Beispiel erläutert daher einmal, daß die ISTR-Analyse Hinweise auf Umordnungen des genetischen Materials zu geben vermag. Zweitens ist allein aus der Verwendung eines einzigen ISTR-Primerpaars eine Fingerprint-Analyse von Hochleistungssorten möglich. Daraus folgt, daß mit der Verwendung weiterer ISTR-Primerpaare ein eindeutiger sortenspezifischer Fingerprint erhalten werden kann, der als biochemische Charakterisierung der Sorte dient (Sortenschutz).

### Beispiel 3

### Test auf Anwendung bei Tieren: Evidenz für segregierende sowie neu entstehende Marker in Familien

Zum Test auf die generelle Anwendbarkeit der ISTR-Analyse genetischen Materials außerhalb des Pflanzenreichs wurden Tierfamilien untersucht, bei denen der Vater durch kontrollierte Züchtung (in vitro-Fertilisation) bekannt war. Fig. 7 illustriert eine ISTR-Analyse mit dem Primerpaar ISTR5/ISTR-2 an einer Rinderfamilie (Fig. 7A) und an zwei Schaffamilien mit identischem Vater, aber zwei verschiedenen Müttern M1 (Fig. 7B) und M2 (Fig. 7C). Aus beiden Analysen ist ersichtlich, daß 1) Kokosnuß-spezifische ISTR-Primer auch im Tierreich zur Fingerprint-Analyse angewendet werden können, und daß 2) sowohl segregierende Marker (siehe Pfeile in Fig. 7C) als auch individuum-spezifische Marker (siehe Sterne in Fig. 7) durch die ISTR-Analyse zugänglich sind. Einen Hinweis, daß segregierende ISTR-Marker mit wichtigen Phänotypen kosegregieren können, gibt die mit SSM (sexspezifischer Marker) bezeichnete DNA-Bande des prominenten Triplets in Fig. 7B, C: Diese Bande ist im Vater, nicht jedoch in den beiden Müttern vorhanden. Tatsächlich sind die beiden Nachkommen der Familie 1 (Fig. 7B) weiblichen Geschlechts, während die Familie 2 (Fig. 7C) einen männlichen Nachkommen hat. Die Tatsache, daß elterliche Marker nicht in allen Nachkommen vorhanden sind (siehe Pfeil in Fig. 7A) bzw. daß neue Marker entstehen (siehe Steme in Fig. 7), kann als Hinweis interpretiert werden, daß die ISTR-Analyse Rekombinationsereignisse bei Kreuzungen entdecken kann.

### Beispiel 4

### Test auf Anwendung beim Menschen: Evidenz für geschlechts- und individuum-spezifische Polymorphismen

Dieses Beispiel erläutert die Anwendung der ISTR-Analyse im humanen Bereich. Dazu wurden drei Familien I, II und III analysiert, wobei die beiden Kinder der Familien I und II jeweils homozygote (eineiige) Zwillinge waren. Da von vorneherein nicht zu erwarten war, daß ISTR-Primer DNA-Polymorphismen bei eineiigen Zwillingen entdecken können (hochpolymorphe Mikrosatellitenprimer zeigen keine Unterschiede; Haas, Institut für Rechtsmedizin, Universität Giessen; persönliche Mitteilung), wurden 6 verschiedene ISTR-Primerpaare getestet. Bei allen 6 Analysen sind DNA-Polymorphismen sichtbar, und zwei der ISTR-Analysen mit den Primerpaaren ISTR6/ISTR-1 und ISTR6/ISTR-2 sind in Fig. 8 dargestellt. Die Analyse mit dem Primerpaar ISTR6/ISTR-1 (Fig. 8A) ist bemerkenswert für die Vielzahl von polymorphen DNA-Banden, die individuumspezifisch sind und selbst bei den beiden Paaren von eineiigen Zwillingen der Familien I und II eine eindeutige Charakterisierung des individuellen Menschen zulassen. Dies trifft ebenfalls für die in Fig. 8B mit dem Primerpaar ISTR6/ISTR-2 durchgeführte ISTR-Analyse zu, auch wenn die Anzahl der polymorphen Banden geringer ist. Bemerkenswerterweise findet sich hier unter den neuen Polymorphismen eine DNA-Bande (SSM in Fig. 8B), die nur in den drei Vätern, nicht jedoch in den drei Müttern und den fünf Kindern auftritt. Tatsächlich könnte es sich hier, wie im Beispiel 3 für die Schaffamilien erwähnt, um einen sexspezifischen Marker handeln, da alle 5 Kinder weiblichen Geschlechts sind und somit eine strikt geschlechtsspezifische Segregation bei insgesamt 11 Individuen gegeben ist.

### Beispiel 5

### Nachweis von ISTR-Fingerprints bei Wein

Für diesen Versuch, der in Figur 9 dargestellt ist, wurde das Primerpaar ISTR5/ISTR-2 (siehe Tabelle 1) verwendet. Als zu untersuchende DNAs wurden die genomischen DNAs von 19 Vitis vinifera L. Pflanzen einschließlich 13 vermuteter "Sangiovese" Genotypen und 6 "gefärbte" Ecotypen verwendet, deren Früchte von Bedeutung für die intensive Rotfärbung des Weines sind. Aus Figur 9 ist ersichtlich, daß eine große Anzahl polymorpher DNA-Fragmente erhalten wurden. Obwohl die Variabilität am größten in den "gefärbten" Ecotypen ist, konnten die ISTR-Analyse außerdem einen hohen Anteil von Polymorphismen in den "Sangiovese"-Genotypen feststellen. Diese Unterschiede sind möglicherweise auf die polyclonale Herkunft vieler Weinkultivare zurückzuführen. Daher zeigt auch dieses Beispiel, daß die ISTR-Analyse eine effiziente und sensitive Methode für die Untersuchung der genetischen Diversität innerhalb von Ecotypen und für die Identifizierung von einzelnen Clonen einsetzbar ist.

### Beispiel 6

### Anwendung des ISTR-Fingerprints bei Mikroorganismen

Als Beispiel für die Anwendung der ISTR-Technologie auf Mikroorganismen dienten Isolate des Pilz Phytophthora palmivora, der auf Kokospalmen lethale Erkrankungen ("bud rot") hervorruft. Es wurde hier ein besonders diffiziles Beispiel für die Anwendung einer DNA-Marker-Technologie gewählt, für das eigentlich aufgrund der begrenzten genetischen Diversität nur wenige Polymorphismen erwartet wurden, da es sich in allen Fällen um P. palmivora-Isolate handelte, die zudem ausschließlich in den Philippinen isoliert wurden und auch hier überwiegend lokal begrenzt waren (die Isolate stammten vorwiegend von der Insel Mindanao).

Es wurden je 1µg DNA von achtzehn P. palmivora-lsolaten aus den Philippinen in einer Standard-PCR-Reaktion mit der Primer-Kombination ISTR5/ISTR-2 amplifiziert, die Produkte in bekannter Weise durch PAGE auf einem 4%igen Polyacrylamid-Gel aufgetrennt und die einzelnen Banden durch Autoradiographie sichtbar gemacht. Fig. 10 zeigt das Ergebnis dieser Analyse. Bereits durch die Gelanalyse (Fig. 10A) werden mit einer einzigen ISTR-Primer-Kombination eine Fülle polymorpher DNA-Fragmente sichtbar. Dreißig dieser Banden wurden nach bekannten Verfahren der Cluster-Analyse ausgewertet zu Phenogrammen nach der UPGMA-Methode (SAHN-Clustering; Fig. 10B) und durch PCA (principal coordinate analysis; Fig.10C). Die erhaltenen Daten stimmen gut mit der anhand von RAPD-DNA-Marker-Analysen vorgenommenen Klassifizierung dieser Isolate überein.

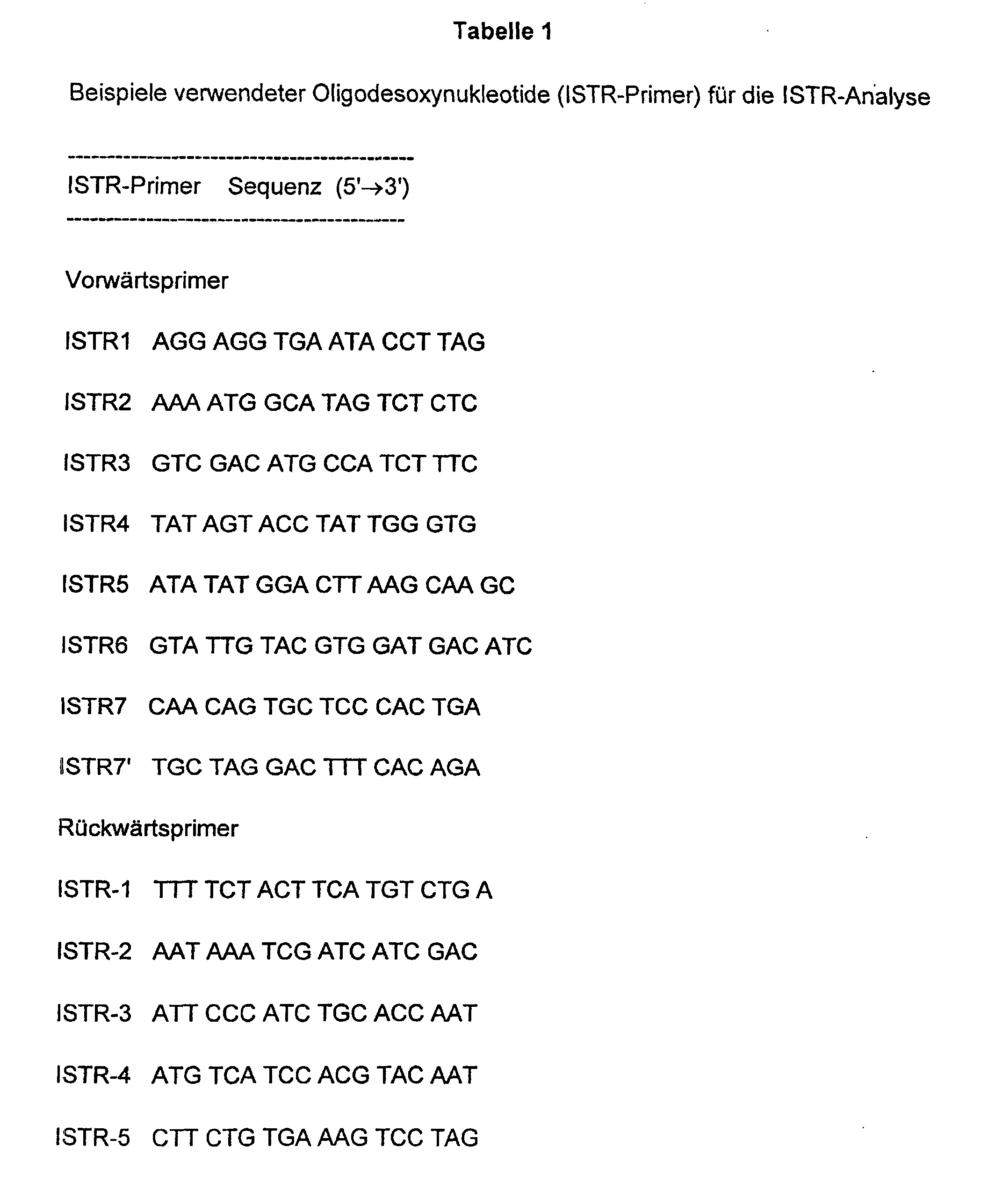

## Patentansprüche

1. Verwendung eines Primers oder Primerpaares, wobei der Primer oder das Primerpaar einer DNA-Sequenz der DNA-Fragmente der copia-ähnlichen Elemente entspricht, die in der nach Restriktion isolierter genomischer DNA aus Kokosnuß (Cocos nucifera L.) mit dem Restriktionsenzym EcoRI und Auftrennung im Agarosegel als zwei Fragmente, jeweils mit 1.3 und 1.4 Kilobasen, sichtbar sind, zur DNA-Fingerprint-Analyse von Menschen, Tieren, Pflanzen oder Mikroorganismen, und wobei
(a) das Tier aus dem Tierreich mit allen Unterreichen, vorzugsweise die Metazoen, darin enthalten die Unterstämme der Vertebraten, dabei vorzugsweise die Klasse der Säugetiere, darin enthalten insbesondere die Familie der Hominiden und die Familie der Bovidae, darin enthalten die Spezies Bovis taurus und Ovis aries, sowie alle Rassen und Varietäten, die sich aus der entsprechenden Spezies ableiten lassen, stammt;
(b) die Pflanze aus dem Unterreich der Mycobionta, aus der Familie der Poaceae mit ihren Vertretern der Spezies Hordeum vulgare und Zea mays, oder aus der Klasse der Dicotyledonae mit ihren Familien, z.B. der Solanaceae und ihrem Vertreter der Spezies Solanum tuberosum, Nicotiana tabacum, Petunia hybrida, oder z.B. die Familie der Brassicaceae mit ihrem Vertreter der Spezies Brassica napus oder die Familie der Chenopodiaceae mit ihrem Vertreter Beta vulgaris oder die Familie der Vitaceae mit ihrem Vertreter Vitis vinifera sowie alle Varietäten und Sorten, die sich von der entsprechenden Spezies ableiten lassen, stammt;
(c) Mikroorganismen prokaryotische Mikroorganismen, dabei vorzugsweise Gram-positive Bakterien wie z.B. Milchsäurebakterien, Sarcina und Coryneforme Bakterien und Gram-negative Bakterien wie z.B. Neisseria und Enterobakterien, und eukaryotische Mikroorganismen umfassend Pilze, dabei vorzugsweise Phycomyceten wie z.B. Phytophthora und Ascomyceten wie z.B. Hefen umfassen.

2. Verwendung nach Anspruch 1, **dadurch gekennzeichnet, daß** die zu analysierenden DNAs mit dem Primer oder dem Primerpaar durch PCR amplifiziert und nachfolgend der Größe nach auf einem Gel aufgetrennt werden.

3. Verwendung nach Anspruch 2, **dadurch gekennzeichnet, daß** das Gel ein Sequenzgel ist.

4. Verwendung nach Anspruch 2 oder 3, **dadurch gekennzeichnet, daß** als weiterer Schritt ein Southern Blot durchgeführt wird, und die auf die Membran übertragenen DNAs durch Hybridisierung mit einer Sonde sichtbar gemacht werden.

5. Verwendung nach Anspruch 4, **dadurch gekennzeichnet, daß** die Sonde der bzw. das in einem der vorstehenden Ansprüche genannte Primer oder Primerpaar ist.

6. Verwendung nach einem der Ansprüche 1 bis 5, **dadurch gekennzeichnet, daß** der Primer oder das Primerpaar eine Markierung trägt.

7. Verwendung nach Anspruch 6, **dadurch gekennzeichnet, daß** die Markierung eine nicht-radioaktive Markierung, insbesondere Digoxigenin, Biotin, ein Fluoreszenzfarbstoff, ein Farbstoff oder eine radioaktive Markierung, insbesondere ³²P ist.

8. Verwendung nach einem der Ansprüche 1 bis 7, **dadurch gekennzeichnet, daß** der Primer eine der folgenden Sequenzen aufweist:
(Vorwärtsprimer: 5'→3') (Rückwärtsprimer 5'→ 3')

9. Verwendung nach einem der Ansprüche 1 bis 8, **dadurch gekennzeichnet, daß** die Fingerprint-Analyse für Biodiversitätsstudien, Studien zur genetischen Verwandtschaft, taxonomische Studien, und insbesondere in der Rechtsmedizin, der Züchtung, im Sortenschutz, im Genbank-Management, in der Diagnostik, in der Populationsgenetik und für Evolutionsstudien eingesetzt wird.

10. Primer zur Verwendung nach einem der vorgenannten Ansprüche, **dadurch gekennzeichnet, daß** der Primer eine der folgenden Sequenzen aufweist:
(Vorwärtsprimer: 5'→3') (Rückwärtsprimer 5'→ 3')

## Claims

1. Use of a primer or pair of primers, wherein said primer or said pair of primers corresponds to a DNA sequence of DNA fragments of copia-like elements which are visible after restriction of isolated genomic DNA from coconut (Cocus nucifera L.) with the restriction enzyme EcoRI and separation in an agarose gel as two fragments, each with 1.3 and 1.4 kilo bases for DNA-fingerprint analysis of humans, animals, plants or microorganisms, whereby
(a) said animal is derived from the animal kingdom including all subkingdoms, preferably metazoans, containing all subphyla of the vertebrates, preferably the class of mammals, particularly containing the family of the hominids and the family of the bovidae, containing the species Bovis taurus and Ovis aries, as well as all races and varieties derived from the corresponding species;
(b) said plant is derived from the subkingdom of the Mycobionta, from the family of Poaceae including its representatives of the species Hordeum vulgare and Zea mays, or from the class of the Dicotyledonae including its families, for example, the Solanaceae and its representative of the species Solanum tuberosum, Nicotiana tabacum, Petunia hybrida or, for example, the family of the Brassicaceae with its representative of the species Brassica napus or the family of the Chenopodiaceae with its representative Beta vulgaris or the familiy of the Vitaceae with its representative Vitis vinifera as well as all varieties and cultivars derived from the respective species;
(c) microorganisms, prokaryotic microorganisms, particularly Gram-positive bacteria, for example, lactic acid bacteria, Sarcina and Coryneform bacteria and Gram-negative bacteria, for example, Neisseria and enterobacteria and eukaryotic microorganisms comprising fungi, preferably comprising Phycomycetes, for example, Phytophthora and Ascomycetes, for example, yeasts.

2. The use of claim 1 **characterized in that** said DNAs to be analysed are amplified by PCR with said primer or said pair of primers and are subsequently separated on a gel according to their size.

3. The use of claim 2 **characterized in that** said gel is a sequencing gel.

4. The use of claim 2 or 3 **characterized in that** a Southern Blot is carried out as further step and the DNAs transferred to the membrane are visualized by hybridisation with a probe.

5. The use of claim 4 **characterized in that** said probe is said primer or said pair of primers referred to in any of the preceding claims.

6. The use of any one of claims 1 to 5 **characterized in that** said primer or said pair of primers carries a label.

7. The use of claim 6 **characterized in that** said label is a non-radioactive label, particularly digoxigenin, biotin, a fluorescent dye, a dye or a radioactive label, particularly ³²P.

8. The use of any one of claims 1 to 7 **characterized in that** said primer has one of the following sequences:
(forward primer: 5'→3') (backward primer 5'→3')

9. The use of any one of claims 1 to 8 **characterized in that** said fingerprint analysis is employed for biodiversity studies, studies for genetic relation, taxonomic studies and particularly in forensic medicine, breeding, plant variety protection, gene bank management, diagnosis, population genetics and evolution studies.

10. A primer for the use of any one of the preceding claims **characterized in that** said primer has one of the following sequences:
(forward primer: 5'→3') (backward primer: 5'→3')

## Revendications

1. Utilisation d'une amorce ou d'une paire d'amorces, l'amorce ou la paire d'amorces correspondant à une séquence d'ADN des fragments d'ADN des éléments de type copie qui sont visibles dans l'ADN génomique de noix de coco (*Cocos nucifera L.*) isolé après restriction par l'enzyme de restriction *Eco* RI et séparation sur gel d'agarose sous forme de deux fragments, ayant respectivement 1,3 et 1,4 kilobases, pour l'analyse de profil (*fingerprint*) d'ADN d'individus humains, d'animaux, de plantes ou de micro-organismes, et
(a) l'animal étant issu du règne animal comportant tous les sous-règnes, de préférence les métazoaires, comprenant les sous-embranchements des vertébrés, parmi ceux-ci de préférence la classe des mammifères, comprenant en particulier la famille des hominidés et là famille des bovidés, comprenant les espèces *Bovis tourus* et *Ovis aries*, ainsi que toutes les races et variétés qui peuvent être issues des espèces correspondantes ;
(b) la plante étant issue du sous-règne des mycobiontes, de la famille des poacées, avec ses représentants appartenant aux espèces *Hordeum vulgare* et *Zea mays*, ou de la classe des dicotylédones avec leurs familles, par exemple des solanacées et leurs représentants des espèces *Solanum tuberosum*, *Nicotiana tabacum*, *Pétunia hybrida*, ou par exemple la famille des brassicacées avec ses représentants de l'espèce *Brassica napus* ou la famille des chénopodiacées avec son représentant *Beta vulgaris* ou la famille des vitacées avec son représentant *Vitis vinifera* ainsi que toutes les variétés qui peuvent dériver des espèces correspondantes ;
(c) les micro-organismes comprenant des micro-organismes procaryotes, parmi ceux-ci de préférence des bactéries à Gram positif, comme par exemple, les bactéries lactiques, *Sarcine* et les bactéries corynéformes, et des bactéries à Gram négatif, comme par exemple, *Neisseria* et les entérobactéries, et des micro-organismes eucaryotes comprenant les champignons, parmi ceux-ci de préférence les phycomycètes comme par exemple *Phytophthora* et les ascomycètes comme par exemple les levures.

2. Utilisation selon la revendication 1, **caractérisée en ce que** les ADN à analyser sont amplifiés par PCR avec l'amorce ou la paire d'amorces et sont ensuite séparés en fonction de la taille sur un gel.

3. Utilisation selon la revendication 2, **caractérisée en ce que** le gel est un gel de séquençage.

4. Utilisation selon la revendication 2 ou 3, **caractérisée en ce qu'**on effectue en tant qu'autre étape une analyse Southern blot et les ADN transférés sur la membrane sont rendus visibles par hybridation avec une sonde.

5. Utilisation selon la revendication 4, **caractérisée en ce que** la sonde est l'amorce ou la paire d'amorce citée dans l'une des revendications précédentes.

6. Utilisation selon l'une quelconque des revendications 1 à 5, **caractérisée en ce que** l'amorce ou la paire d'amorces porte un marqueur.

7. Utilisation selon la revendication 6, **caractérisée en ce que** le marqueur est un marqueur non radioactif, en particulier la digoxygénine, la biotine, un colorant fluorescent, un colorant ou un marqueur radioactif, en particulier ³²P,

8. Utilisation selon l'une quelconque des revendications 1 à 7, **caractérisée en ce que** l'amorce présente l'une des séquences suivantes :
(amorces directes : 5'→3') (amorces inverses 5'→3')

9. Utilisation selon l'une quelconque des revendications 1 à 8, **caractérisée en ce que** l'analyse de profil *fingerprint* est utilisée pour des études de bio-diversité, des études pour la détermination du lien de parenté génétique, des études taxonomiques et en particulier en médecine légale, dans la sélection, dans la protection des variétés, dans la gestion de banques de gènes, en diagnostic, en génétique des populations et pour des études de l'évolution.

10. Amorce pour utilisation selon l'une quelconque des revendications précédentes, **caractérisée en ce que** l'amorce présente l'une des séquences suivantes :
(amorces directes : 5'→3') (amorces inverses 5'→3')
